# DEMANDE DE BREVET EUROPEEN

(11) **EP 3 100 737 A1**
(43) Date de publication de la demande: **07.12.2016**
(21) Numéro de dépôt: 16172740.9
(22) Date de dépôt: 02.06.2016
(51) Int. Cl.: A61K 33/08, A61K 33/10

(54) **COMPLÉMENT ALIMENTAIRE COMPRENANT UN MÉLANGE D'OXYDE DE MAGNÉSIUM ET DE CARBONATE DE MAGNÉSIUM**

(30) Priorité: 03.06.2015 FR 1555051
(71) Demandeur: Densmore et Cie, 98002 Monaco (MC)
(72) Inventeur: Caron, Philippe, 06000 Nice (FR)
(74) Mandataire: Richaud, Fabien

(57) **Abrégé**

Complément alimentaire administrable par voie orale comprenant un mélange d'oxyde de magnésium et de carbonate de magnésium.

## Description

La présente invention concerne le domaine des compléments alimentaires à usage humain ou animal, de préférence à usage humain.

Le magnésium est un élément chimique, de symbole Mg et de numéro atomique 12. Le magnésium est un nutriment minéral essentiel au bon fonctionnement des organismes humains ou animaux. Il participe à plus de 300 réactions métaboliques dans le corps humain. Il agit en association étroite avec d'autres ions tels que le sodium, le potassium et le calcium, avec lesquels il doit rester en équilibre dans l'organisme. Le magnésium contribue notamment à la transmission nerveuse et à la relaxation musculaire après contraction, ce qui est vital pour la fonction cardiaque. En particulier, il est essentiel au maintien d'un rythme cardiaque régulier, au métabolisme des lipides, ainsi qu'à la régulation du taux de sucre sanguin et de la tension artérielle. D'un point de vue thérapeutique, le magnésium est traditionnellement utilisé dans des traitements thérapeutiques aussi divers que :
- la prévention de certains troubles cardiovasculaires,
- la prévention du diabète de type Il et ses complications,
- le soulagement des symptômes du syndrome prémenstruel,
- la prévention des crises d'asthme,
- la prévention de la récurrence des calculs rénaux,
- l'amélioration des performances sportives,
- le soulagement des crampes dans les jambes durant la grossesse,
- le traitement du trouble du déficit d'attention avec hyperactivité (dont l'acronyme est TDAH), et
- la contribution à la prévention de l'ostéoporose (liste non-exhaustive).

Le magnésium possède d'autres usages thérapeutiques reconnus ou potentiels qui relèvent d'un suivi médical spécifique. Il est, par exemple, employé en injections intraveineuses ou intramusculaires pour l'éclampsie et la pré-éclampsie, le tétanos, les troubles du rythme cardiaque, la neuropathie associée au cancer ou encore utilisé comme laxatif ou antiacide. En outre, de par son action relaxante sur les muscles lisses, dilatante sur les vaisseaux et normalisatrice sur la conduction nerveuse, le magnésium agirait comme un neuroprotecteur au niveau cérébral et posséderait ainsi des effets bénéfiques en matière de fonctions cognitives et notamment de mémorisation. Cet effet neuroprotecteur apparaît être lié au fait que le magnésium possède un effet inhibiteur calcique par inhibition compétitive, s'opposant à l'entrée massive de calcium dans les cellules neuronales en souffrance. Cette entrée massive de calcium dans les cellules neuronales en souffrance active la calcineurine, laquelle entraîne, à son tour, le relargage de facteurs apoptotiques comme les caspases, libérées par les mitochondries dans le cytoplasme (le calcium cytoplasmique stimulant également la production d'oxyde nitrique). Il résulte de cette entrée massive de calcium dans les cellules neuronales en souffrance une dégénérescence des neurones et des cellules ganglionnaires rétiniennes. De manière plus spécifique, en ce qui concerne l'effet neuroprotecteur du magnésium, il a été démontré que le magnésium réduisait l'apoptose neuronale secondaire aux lésions d'ischémie-reperfusion (ce qui pourrait être en lien avec un effet freinateur de l'expression des protéines apoptotiques caspase-3 et bax, tel qu'indiqué dans Zhou et al. [1]).

Une étude récente réalisée par Aydin et al. en 2010 [2] a également démontré l'effet bénéfique du magnésium sur l'amélioration du champ visuel. Plus précisément, cette publication établit qu'un apport en magnésium améliore le champ visuel chez les patients atteints de glaucome à pression normale, et ce sans modification du débit sanguin oculaire. En outre, une autre publication, à savoir Gaspar et al. [3], établit que le magnésium améliore la circulation périphérique et semble avoir un effet bénéfique sur le champ visuel des patients atteints de glaucome avec vasospasme, et ce sans conséquence sur la pression artérielle ou encore la fréquence des pulsations cardiaques.

Or, parmi les nombreux troubles directement liés au vieillissement, ceux touchant les principales fonctions du cerveau comme la mémoire ou les organes des sens, comme l'oeil, sont fréquemment rencontrés, faisant également du magnésium un bon candidat dans le cadre de la prévention des effets neurologiques associés au vieillissement.

Les apports journaliers recommandés (AJR) - ou apports nutritionnels recommandés (ANR)
- en magnésium généralement admis par la littérature sont résumés dans le Tableau 1 infra :

**Tableau 1 : Apport nutritionnel recommandé en magnésium (source : Institute of Medicine, Food and Nutrition Board, 1997) [4]**

| **Apport nutritionnel recommandé en magnésium** | |
|---|---|
| **Age** | **mg/jour** |
| **de 1 à 3 ans** | 80 |
| **de 3 à 8 ans** | 130 |
| **de 9 à 13 ans** | 240 |
| **Garçon, de 14 à 18 ans** | 410 |
| **Fille, de 14 à 18 ans** | 360 |
| **Homme, de 19 à 30 ans** | 400 |
| **Femme, de 19 à 30 ans** | 310 |
| **Homme, 31 ans et plus** | 420 |
| **Femme, 31 ans et plus** | 320 |
| **Femme enceinte** | 18 ans et moins : 400 |
| | de 19 à 30 ans: 350 |
| | 31 ans et plus : 360 |
| **Femme qui allaite** | 18 ans et moins : 360 |
| | de19à30ans:310 |
| | 31 ans et plus : 320 |

Il était traditionnellement admis que les apports journaliers recommandés en magnésium pouvaient être obtenus via une alimentation comprenant des aliments riches en magnésium, tels que des céréales entières, des noix, des graines, des légumineuses, des légumes « à feuilles vert foncé » (par exemple des épinards) etc. Or, des données européennes et nord-américaines **[5]** et **[6]** indiquent que les apports alimentaires en magnésium sont souvent en deçà des apports nutritionnels recommandés, en particulier chez les personnes âgées, lesquelles devraient a contrario bénéficier, entre autres, des effets neuroprotecteurs du magnésium, notamment en liaison avec les processus cognitifs (en particulier la mémorisation) et la vision.

Au demeurant, les carences en magnésium chez les individus humains peuvent être dues - au moins en partie - à d'autres causes, ou accentuées par ces dernières, lesdites causes étant notamment :
- la prise à long terme de certains médicaments qui augmentent les pertes en magnésium dans l'urine, tels que certains diurétiques (diurétiques de l'anse, diurétiques thiazidiques), antibiotiques (par exemple dentamicine et amphotéricine) et certains immunosuppresseurs comme la cyclosporine ;
- une mauvaise absorption intestinale du magnésium causée, par exemple, par la maladie de Crohn, la maladie coeliaque ou une chirurgie intestinale ;
- l'alcoolisme ;
- la prise de contraceptifs oraux, d'oestrogènes et de cisplatine (médicament anticancéreux) ; ou encore
- la consommation excessive d'autres suppléments minéraux (dans la mesure où les minéraux interagissent les uns avec les autres, un excès de manganèse ou de potassium, peut, par exemple, entraîner une carence/un déficit en magnésium).

Afin de pallier ces carences/déficits en magnésium, de nombreux compléments alimentaires *per os* ont été développés et commercialisés. Pour que le magnésium soit (biologiquement) assimilable par un organisme humain ou animal (en d'autres termes, bioassimilable ou bioassimilable), il est nécessaire que le magnésium soit apporté à l'organisme sous forme hydrosoluble (soluble en milieu aqueux), de sorte que le magnésium puisse se solubiliser dans les liquides biologiques (tels que le sang et/ou les liquides extravasculaires), lors du processus de digestion. C'est pourquoi, parmi les différents sels de magnésium existants, plus ou moins solubles dans les liquides biologiques après une administration par voie orale, l'on tend à privilégier les sels de magnésium connus pour être les plus solubles en milieu aqueux comme les sels de l'acide citrique, de l'acide gluconique ou de l'acide glycérophosphorique, lesquels sont généralement considérés comme étant bien absorbés et bien tolérés au niveau du tube digestif, tandis que les formes connues pour être moins solubles en milieu aqueux, telles que l'oxyde de magnésium (MgO ; n° CAS : 1309-48-4) ou le carbonate de magnésium (MgCO₃ ; n° CAS : 546-93-0) sont traditionnellement considérées comme moins bien assimilées et tolérées au niveau digestif. L'oxyde de magnésium ou le carbonate de magnésium sont d'ailleurs décrits dans la littérature en lien avec leurs propriétés antiacides, notamment dans le cadre du traitement d'acidités gastriques et brûlures d'estomac chez des patients humains. Ainsi, le brevet US 5,320,852 divulgue un comprimé biconvexe spécifique comprenant, en tant qu'agent antiacide, un antiacide sélectionné dans le groupe comprenant notamment l'oxyde de magnésium et le carbonate de magnésium (aucun comprimé comprenant l'un ou l'autre de ces composés n'étant d'ailleurs spécifiquement exemplifié). De manière similaire, le document US 6,589,507 divulgue un médicament antiacide comprenant une préparation pharmaceutique effervescente *per os* (formulée de préférence sous forme de comprimé à mâcher), adaptée pour réguler l'hyperacidité des sucs gastriques, ladite préparation comprenant un (i) composé « antiacide » ou un mélange de composés « antiacides » choisi(s) parmi les composés suivants : l'hydroxyde de magnésium, l'oxyde de magnésium, le carbonate de magnésium, le silicate de magnésium, l'hydroxyde d'aluminium, le phosphate d'aluminium et le silicate de magnésium et d'aluminium. Outre ce composé « antiacide » ou ce mélange de composés « antiacides », cette préparation pharmaceutique effervescente requiert un mélange complexe de constituants à savoir :
(ii) un mélange effervescent libérant du CO2,
(iii) un agent tensioactif polymère choisi parmi les poloxamères en tant qu'agent de formation de mousse (« foam-forming agent », en langue anglaise) ou un mélange d'agents tensioactifs de ce type, et
(iv) un polymère de gonflement et de formation d'un gel (« swelling and gel-forming polymer », en langue anglaise), choisi parmi la gomme adragante, le galactomannane, la méthyl-cellulose, l'hydroxypropylmethyl cellulose ou l'hydroxybutylmethyl cellulose), différent du composant (iii), ou un mélange de polymères de ce type, et, le cas échéant,
(v) des substances auxiliaires classiques.

Le brevet britannique GB 392 (n° de publication : 191300392 (A)), en date de 1913, décrit, quant à lui, une composition comprenant du carbonate de calcium précipité et du « super-oxyde » de magnésium destinée à traiter les brûlures d'estomac.

Toutefois, un des problèmes liés à l'utilisation des sels de l'acide citrique, de l'acide gluconique ou de l'acide glycérophosphorique, et, de manière générale, des sels de magnésium considérés comme étant plus « hydrosolubles », réside dans leur faible teneur en magnésium (entre 5% et 15%). Afin de garantir un apport suffisant en magnésium à un individu humain ou animal, de préférence humain, à savoir une quantité d'au moins 150 mg d'élément magnésium, il s'avère donc nécessaire d'administrer à cet individu une unité de prise (dose unitaire) très importante. En d'autres termes, ceci aboutit à la préparation de compositions, notamment de compléments alimentaires, de masse et/ou de volume plus important(e)(s) que nécessaire. Ceci est particulièrement significatif pour les formes galéniques solides (plus simplement dénommées « formes solides »), pour lesquelles il s'avère nécessaire d'administrer à un individu humain ou animal, de préférence humain, une composition solide dont la masse représente plus de 1000 mg de sels de magnésium considérés comme étant les plus « hydrosolubles » (cf. supra), afin de garantir un apport d'au moins 150 mg d'élément magnésium au sujet, tel qu'indiqué précédemment (la teneur en élément magnésium de ces sels étant seulement de 5% à 15%, comme mentionné ci-dessus). Qui plus est, cette masse de 1000 mg ne prend pas en compte les additifs technologiques de dilution, indispensables à une présentation finale du produit et qui représentent généralement une fraction comprise entre 30% et 80% de la composition finale. Compte tenu de ces spécificités, une forme solide destinée à garantir au consommateur un apport en magnésium suffisant, pourrait aisément avoir une masse de plus de 3000 mg, ce qui ne parait pas envisageable au regard du critère essentiel de facilité de prise du produit. En effet, il est connu de l'homme du métier que les formes solides unitaires, par exemple de type comprimé, de masse supérieure à 1000 mg sont trop volumineuses et, par conséquent, très difficilement ingérables par le consommateur - en particulier par le consommateur âgé - impactant *de facto,* de manière très négative, l'observance de la cure associée, en particulier pour une cure « au long cours ».

En résumé, il existe donc un besoin de mettre au point une composition, en particulier sous forme de complément alimentaire, permettant un apport important en élément magnésium à l'organisme tout en ayant une masse et/ou un volume réduit(e)(s).

Dans le cadre de ses recherches, et contre toute attente, la Demanderesse a découvert qu'il était possible de répondre à ce besoin en combinant deux sels de magnésium considérés comme a priori peu solubles - voire insolubles - en milieu aqueux. Par conséquent, l'invention a pour objet un complément alimentaire (composition nutritionnelle) administrable par voie orale à un individu humain ou animal, de préférence humain, ledit complément alimentaire comprenant un mélange d'oxyde de magnésium et de carbonate de magnésium, de préférence en tant que seule source de magnésium.

Ainsi, malgré le fait que l'oxyde de magnésium et le carbonate de magnésium soient habituellement considérés comme deux espèces difficilement solubles - voire insolubles - en milieu aqueux, la Demanderesse a découvert, de manière surprenante, que le mélange/combinaison de ces deux espèces permettait de mettre au point un complément alimentaire garantissant un apport important en élément magnésium à l'organisme tout en ayant une masse et/ou un volume réduit(e)(s).

Sans être lié par la théorie, il semble que cet effet technique aussi avantageux que surprenant soit dû à l'excellente cinétique de dilution, en milieu gastrique, dudit mélange d'oxyde de magnésium et de carbonate de magnésium. En effet, toujours sans être lié par la théorie, le temps de dissolution du magnésium contenu dans le susdit mélange au sein du milieu stomacal apparaît être, en moyenne, inférieur à 90 minutes. Le magnésium étant ainsi en grande partie (voire en quasi-totalité ou en totalité) dissous dans le milieu stomacal, il peut ensuite être facilement assimilé dans le milieu intestinal, ce critère étant jugé essentiel pour obtenir un complément alimentaire d'une grande efficacité. Outre la teneur importante en élément magnésium, le susdit mélange d'oxyde de magnésium et de carbonate de magnésium s'avère donc être, contre toute attente, une source de magnésium assimilable par l'organisme (en d'autres termes « biologiquement assimilable »), et plus précisément hautement assimilable par un organisme humain ou animal, de préférence par un organisme humain. Par « hautement assimilable », l'on entend, au sens de la présente invention, une absorption de plus de 80% de l'élément magnésium au niveau du tube digestif.

Il ressort de ce qui précède que l'invention peut donc être également formulée comme suit : un complément alimentaire administrable par voie orale à un individu humain ou animal, de préférence humain, comprenant une source de magnésium à haute teneur (concentrée) en élément magnésium (de préférence stable), ladite source de magnésium à haute teneur en élément magnésium étant assimilable, de préférence hautement assimilable, par l'organisme dudit individu, ladite source de magnésium à haute teneur en élément magnésium comprenant (de préférence consistant en) au moins un mélange d'oxyde de magnésium et de carbonate de magnésium. Par source de magnésium « hautement assimilable », l'on entend, tel qu'indiqué précédemment, une source de magnésium qui permet une absorption de plus de 80% de l'élément magnésium au niveau du tube digestif.

Tel qu'indiqué dans la Directive 2002/46/CE du Parlement européen et du Conseil, en date du 10 juin 2002, relative au rapprochement des législations des Etats membres concernant les compléments alimentaires, l'on entend par « complément alimentaire », une denrée alimentaire dont le but est de compléter le régime normal et qui constitue une source concentrée de nutriments ou d'autres substances ayant un effet nutritionnel ou physiologique seuls ou combinés, commercialisés sous forme de doses, à savoir les formes de présentation telles que les gélules, les pastilles, les comprimés, les pilules et autres formes similaires, ainsi que les sachets de poudres, les ampoules de liquide, les flacons munis d'un compte-gouttes et les autres formes analogues de préparations liquides ou en poudre destinées à être prises en unités mesurées.

Le complément alimentaire selon la présente invention (également dénommée composition nutritionnelle) est à visée diététique/nutritionnelle. Par conséquent, il convient de mettre en exergue le fait que la portée de la présente invention ne s'étend pas aux médicaments, lesquels sont traditionnellement définis comme toute substance ou composition présentée comme possédant des propriétés curatives ou préventives à l'égard des maladies humaines ou animales, ainsi que toute substance ou composition pouvant être utilisée chez l'homme ou chez l'animal ou pouvant leur être administrée en vue d'établir un diagnostic médical ou de restaurer, corriger ou modifier leurs fonctions physiologiques en exerçant une action pharmacologique, immunologique ou métabolique.

Certains médicaments peuvent comprendre, à titre d'excipients, des formes de magnésium connues jusqu'à présent pour être peu solubles - voire insolubles en milieu aqueux - telles que de l'oxyde de magnésium et/ou du carbonate de magnésium. A titre d'exemple de médicaments comprenant de telles formes en tant qu'excipients, l'on peut notamment citer :
- les formulations divulguées dans la publication « Khaled K A » [7] qui comprennent de l'hydrochlorothiazide (dont l'acronyme est "HCT" ou encore "HCTZ"), molécule utilisée comme médicament diurétique dans la classe des thiazidiques, notamment dans le traitement de l'hypertension artérielle ;
- la spécialité dénommée « Diloran » (*Rona, UK*)*,* décrite dans le document « Reynolds et al.» **[8],** comprenant, en tant que principe actif pharmaceutique, de la "diméthicone activée" et, en tant qu'excipients, notamment de l'oxyde de magnésium et du gel d'aluminium hydroxyde et de carbonate de magnésium codesséchés ;
- la forme pharmaceutique *per os* divulguée au paragraphe [0013] de la demande de brevet US 2005/220865, laquelle consiste en un mélange granulaire adapté pour se désintégrer rapidement après administration orale, ledit mélange granulaire comprenant deux types de granules, un type de granules comprenant notamment de l'oxyde de magnésium et du carbonate de magnésium et un deuxième type de granules, en mélange avec le premier, comprenant un principe actif pharmaceutique à savoir l'acétaminophène (également connu sous la dénomination "paracétamol"), ce principe actif pharmaceutique étant un célèbre antalgique et antipyrétique.

Il convient d'ailleurs de noter que les formes de magnésium connues pour être *a priori* peu solubles - voire insolubles - en milieu aqueux (telles que l'oxyde de magnésium et/ou le carbonate de magnésium), lorsqu'elles sont utilisées en tant qu'excipients dans des médicaments, représentent généralement un faible pourcentage en poids de la forme galénique obtenue *in fine* (cf. « Khaled K A » **[7]**, page 41, dernier paragraphe, à page 42, premier paragraphe, où il est indiqué qu'afin de préparer des comprimés de taille raisonnable, le pourcentage de carbonate de magnésium et/ou d'oxyde de magnésium a été maintenu aux environs ≤ de 7% du poids du comprimé (formules 3, 4 et 7)).

Bien évidemment, eu égard à la caractéristique essentielle selon laquelle le complément alimentaire objet de la présente invention est à visée diététique/nutritionnelle, des médicaments, tels que ceux mentionnés supra, comprenant des substances chimiques ou biologiques à action pharmacologique, immunologique ou métabolique et de l'oxyde de magnésium et/ou du carbonate de magnésium en tant qu'excipient(s) sont exclus *de facto* de la portée de la présente invention.

De préférence, le complément alimentaire selon la présente invention comprend au moins 10% en poids, de préférence au moins 20% en poids, avantageusement au moins 30% en poids, et de manière préférée au moins 40% en poids, du mélange d'oxyde de magnésium et de carbonate de magnésium par rapport au poids total du complément alimentaire, le but étant de maximiser l'apport en élément magnésium à l'organisme tout en proposant un complément alimentaire administrable par voie orale ayant une masse et/ou un volume réduit(e)(s).

Selon un mode de réalisation particulier, le complément alimentaire selon l'invention est sous forme liquide, et se présente, de préférence, sous forme d'ampoule(s) ou de flacon(s) éventuellement muni(s) d'au moins un compte-gouttes ou d'au moins une pipette.

Selon un mode de réalisation particulièrement préféré de la présente invention, le complément alimentaire est sous forme solide, de préférence sous forme non-effervescente. Avantageusement, toujours selon ce mode de réalisation particulièrement préféré de la présente invention, ledit complément alimentaire sous forme solide, de préférence non-effervescente, est sous forme de dose unitaire, avantageusement sous forme de comprimé. Dans ce dernier cas, le comprimé est, de préférence, un comprimé dit « classique » (ou « standard »), à savoir un comprimé non enrobé (que l'on administre aux sujets au moyen d'apport d'eau exogène, comme, par exemple, un verre d'eau).

En effet, il s'avère généralement avantageux d'opter pour une forme galénique solide (plus simplement dénommée « forme solide »), en raison d'une meilleure stabilité des nutriments, en particulier du magnésium, en milieu solide qu'en milieu liquide. Tel qu'indiqué précédemment, eu égard à la faible teneur en magnésium des sels de magnésium connus pour être solubles en milieu aqueux (tels que les sels de l'acide citrique, de l'acide glycolique ou de l'acide glycérophosphorique), afin de garantir un apport suffisant en élément magnésium à un individu (à savoir au moins 150 mg d'élément magnésium), une composition sous forme solide comprenant tout ou partie des sels solubles en milieu aqueux susvisés aurait une masse de plus de 1000 mg, cette masse ne prenant pas en compte les additifs technologiques de dilution qui sont indispensables à une présentation finale du produit et qui représentent généralement une fraction comprise entre 30% et 80% de la composition finale. En prenant en compte ces paramètres, la composition solide finale aurait une masse de plus de 3000 mg et serait, par conséquent, très volumineuse et donc *de facto* difficilement ingérable par le consommateur, en particulier par le consommateur âgé. Bien qu'il soit, en théorie, possible de fractionner cette forme solide unitaire, par exemple en trois sous-doses d'environ 1000 mg chacune, ceci n'est pas envisageable d'un point de vue pratique pour la bonne et simple raison que le nombre total de prises serait dès lors beaucoup trop élevé et représenterait une contrainte trop importante pour le bon suivi quotidien de la cure (observance de la cure), notamment pour les populations âgées qui suivent généralement plusieurs traitements médicamenteux et/ou cures nutritionnelles de manière concomitante.

A l'inverse, l'utilisation d'un mélange d'oxyde de magnésium et de carbonate de magnésium permet la mise au point d'un complément alimentaire selon l'invention, sous forme solide (préférablement sous forme de comprimé, avantageusement sous forme de comprimé « classique », tel qu'indiqué supra), dont la taille et le volume sont parfaitement adaptés à une ingestion régulière *per os* chez tout type de population, y compris chez les populations les plus âgées. La présente invention permet, par conséquent, de garantir au consommateur un apport suffisant en élément magnésium dans une forme solide unitaire de taille et de volume compatibles avec une ingestion régulière chez tous types de consommateurs, même les plus âgés.

Le complément alimentaire selon la présente invention peut se présenter sous forme de poudre (forme pulvérulente), de gélule, de granules ou de comprimé ; la forme « comprimé » apparaissant être la plus appropriée, dans la mesure où l'étape de compaction a pour effet de réduire le volume, facilitant encore d'avantage l'ingestion du complément alimentaire par les différentes populations de consommateurs (y compris les populations les plus âgées).

Selon un aspect avantageux du susdit mode de réalisation particulièrement préféré, le complément alimentaire sous forme solide, de préférence sous forme de dose unitaire solide (telle qu'un comprimé), a une masse inférieure à environ 1 g (1 gramme), de préférence inférieure à 1 g. En effet, et tel qu'indiqué précédemment, une forme solide de cette masse (par exemple un comprimé de cette masse) est facilement ingérable par tout type de consommateur, et ce au cours d'une cure à moyen ou long terme, ce qui s'avère particulièrement avantageux. Plus précisément, compte tenu du fait qu'il est généralement recommandé de prendre a *minima* une dose unitaire quotidienne pendant plusieurs semaines, il est donc important de réduire au maximum la masse - et donc le volume associé - de la dose unitaire à ingérer afin de faciliter l'observance de la cure, ceci étant d'autant plus important que le produit s'adresse en particulier à des populations âgées qui peuvent avoir des difficultés de déglutition et pour lesquelles une dose trop importante à ingérer représenterait un handicap sérieux - voire un risque sanitaire - qui pourrait nuire au bon suivi de la cure.

De préférence, le complément alimentaire du susdit mode de réalisation particulièrement préféré, avantageusement sous forme de comprimé, comprend :
- un ou plusieurs additif(s) technologique(s) de compression tel(s) qu'au moins un diluant, au moins un lubrifiant, au moins un agent d'écoulement et/ou au moins un désintégrant,
- éventuellement au moins une substance naturelle ou synthétique telle qu'au moins un colorant ou au moins un arôme.

De préférence, et quel que soit le mode de réalisation envisagé, le mélange d'oxyde de magnésium et de carbonate de magnésium comprend au moins 20% en poids, de préférence plus de 20% en poids, préférablement au moins 30% en poids, avantageusement entre 30% et 75% en poids, et de manière particulièrement préférée entre 40% et 75% en poids, de carbonate de magnésium (le reste représentant, bien évidemment, de l'oxyde de magnésium). En effet, la Demanderesse a découvert, contre toute attente, que ce rapport en poids spécifique, entre deux sels de magnésium considérés jusqu'à présent comme peu solubles - voire insolubles - en milieu aqueux, permettait de réduire au maximum la masse et/ou le volume du complément alimentaire selon l'invention (autorisant *de facto* son administration à tous types de sujets, y compris les sujets âgés et/ou ceux atteints de dysphagie) tout en permettant un apport optimal en élément magnésium à l'organisme du sujet concerné.

Selon un mode de réalisation préféré, l'oxyde de magnésium et/ou le carbonate de magnésium, de préférence l'oxyde de magnésium et le carbonate de magnésium du susdit mélange est/sont sous forme pulvérulente ou granulé dans la composition de base servant à la préparation du complément alimentaire selon l'invention, afin de faciliter l'étape de compression.

Tel qu'indiqué précédemment, le magnésium est également un bon candidat dans le cadre de la prévention des effets neurologiques associés au vieillissement, parmi lesquels figurent les troubles de la mémoire et de la vision. En effet, le cerveau en tant que tel n'échappe pas au vieillissement. L'inflammation, les radicaux libres ou les changements hormonaux sont tous des mécanismes associés au vieillissement du tissu cérébral. Parmi les fonctions cognitives qui déclinent graduellement avec l'âge, la mémoire est l'une des plus souvent citées, en particulier la mémoire dite incidentelle, laquelle permet de retenir automatiquement et pratiquement sans effort. En vieillissant, il devient donc plus difficile de retenir de nombreux détails, sauf à faire un effort conscient.

La mémoire de travail, qui nous permet de retenir, durant quelques secondes, un numéro de téléphone ou de suivre le fil d'une conversation, est aussi fréquemment affectée par l'âge. La vitesse de traitement plus lente qui découle du vieillissement cérébral semble faire disparaître des informations de la mémoire de travail avant même qu'elles n'aient pu être consolidées dans la mémoire à long terme. Cette baisse de performance de la mémoire de travail pourrait donc également expliquer, au moins en partie, l'oubli à plus long terme de nouvelles informations.

En outre, notre capacité à concentrer notre attention sur les aspects pertinents d'un ensemble de données s'amenuise au fil des décennies, cette diminution étant plus significative chez certaines personnes que chez d'autres. La capacité de raisonnement, distraite par des détails inutiles, s'en trouve ainsi ralentie.

En ce qui concerne la vision, une diminution de la vision est fréquemment ressentie avec l'âge, laquelle peut aller jusqu'à la dégénérescence du nerf optique comme dans le cas du glaucome, cette affection pouvant conduire à la cécité. D'une manière générale, le vieillissement des organes sensoriels comme l'oeil entrave donc la saisie correcte des stimuli du monde extérieur et entraine également des répercussions sur l'état cognitif de la personne âgée.

Compte tenu de l'altération de fonctions cérébrales aussi importantes que la vision et la mémoire sous l'effet du vieillissement, la Demanderesse s'est donc intéressée à la mise au point d'un complément alimentaire (composition nutritionnelle) permettant de prévenir, maintenir et/ou améliorer la vision et/ou tout ou partie des fonctions cognitives (en particulier la fonction de mémorisation, notamment en ce qui concerne la mémoire dite incidentelle et/ou la mémoire de travail) chez un sujet âgé. Par « sujet âgé » - ou « consommateur âgé » - l'on entend, au sens de la présente invention, une personne âgée d'au moins 40 ans (étant stipulé que l'ingestion du complément alimentaire selon l'invention est également préconisée chez des consommateurs plus jeunes, notamment en vue de prévenir des troubles en matière de la vision et/ou de fonctions cognitives, en particulier des troubles de la mémorisation). Selon un mode de réalisation préféré, en sus du mélange d'oxyde de magnésium et de carbonate de magnésium (de préférence en tant que seule source de magnésium), la Demanderesse a souhaitée intégrer à son complément alimentaire au moins un extrait de *Ginkgo biloba* et/ou au moins de la cytidine-diphosphate-choline (abrégée en CDP-choline ; également dénommée « citicoline » ; n° CAS 987-78-0), avantageusement les deux.

En effet, L'extrait de *Ginkgo biloba* est notamment connu pour augmenter le débit sanguin cérébral et favoriser la circulation périphérique (en particulier la circulation rétinienne), ce qui contribue à maintenir et améliorer les capacités cognitives (notamment en termes de mémorisation) et la vision chez les sujets âgés. Le *Ginkgo biloba* est un arbre de la famille des ginkgoacées, regroupant des espèces très archaïques. Le *Ginkgo biloba* est utilisé dans la médecine traditionnelle chinoise depuis l'antiquité. L'on utilise en particulier les feuilles de cet arbre, à partir desquelles l'on prépare un extrait qui contient une variété de substances, à savoir notamment des flavonoïdes, des terpénoïdes, des lactones sesquiterpènes, des ginkgolides et des bilobalides.

Concernant la cytidine-diphosphate-choline (CDP-choline ou citicoline), des études ont montré que cette molécule possédait une action bénéfique sur la mémoire et la vision. La citicoline est un intermédiaire de la biosynthèse des phosphatidylcholines, lesquels constituent des phospholipides essentiels aux membranes biologiques. Administrée par voie orale, la citicoline est scindée en choline et en cytidine dans le tube digestif puis recombinée sous forme de citicoline au sein du tissu cérébral.

Même si le rôle nutritionnel du magnésium, de l'extrait de *Ginkgo biloba* et/ou de la CDP-choline en matière de prévention/maintien des fonctions cérébrales, telles que la mémorisation et la vision, est connu de l'homme du métier, il existe cependant des paramètres essentiels à prendre en considération pour que ces nutriments agissent de façon optimale dans l'organisme.

Parmi ces paramètres essentiels figure la dose en nutriment apportée par unité de prise, également dénommée « dose unitaire ». Il ressort des études publiées dans la littérature que les doses moyennes pertinentes apportées pour chacun des trois nutriments présentés ci-dessous sont préférablement a minima de l'ordre de :
- 150 mg de magnésium par unité de prise,
- 40 mg par unité de prise pour l'extrait de *Ginkgo biloba, et*
- 200 mg de CDP-choline par unité de prise.

Outre la dose en nutriment apportée par unité de prise, le caractère assimilable (ou biologiquement assimilable) est un autre paramètre essentiel, dans la mesure où il conditionne l'efficacité du nutriment dans l'organisme. Concernant l'extrait de *Ginkgo Biloba,* celui-ci est très bien décrit dans la littérature pour sa bonne absorption au niveau de la muqueuse intestinale après une administration *per os,* notamment la fraction active terpénique qui renferme les ginkgolides A et B et le bilobalide, lesquels ont une biodisponibilité de 80% à 90 %. La citicoline, quant à elle, est également hautement assimilable au niveau du tube digestif, puisqu'elle est absorbée à plus de 95%. A cet égard, notons que la molécule est successivement transformée dans le tube digestif en choline et cytidine avant d'être synthétisée à nouveau dans le cerveau sous sa forme native de CDP-choline. En revanche, concernant le magnésium, et tel qu'indiqué dans le préambule de la présente demande de brevet, l'homme du métier serait naturellement enclin à privilégier les sels de magnésium les plus solubles en milieu aqueux (hydrosolubles), notamment au sein des liquides biologiques tels que le sang ou les liquides extravasculaires, comme les sels de l'acide citrique, de l'acide gluconique, ou de l'acide glycérophosphorique, en raison de la facilité avec laquelle ces sels de magnésium se solubilisent en milieu aqueux, et notamment au sein des liquides biologiques (tels que le sang ou les liquides extravasculaires). Malheureusement, tel qu'expliqué précédemment, les teneurs moyennes en élément magnésium faibles, voire très faibles (à savoir comprises entre 5% et 15%) de ces sels connus pour être solubles en milieu aqueux, imposent la préparation de doses unitaires sous forme solide de masse et de volume importants incompatibles avec une posologie d'au moins une dose unitaire par jour généralement pendant plusieurs semaines chez tout type de population (y compris les populations les plus âgées). Cette problématique a été exposée en détail supra.

Tel qu'expliqué précédemment, compte tenu de la faible teneur en magnésium des sels dits « hydrosolubles » (tels que les sels de l'acide citrique, de l'acide gluconique ou de l'acide glycérophosphorique), à savoir entre 5% et 15% en moyenne, il ressort que la masse d'un complément alimentaire comprenant du magnésium sous forme de sel(s) dits « hydrosoluble(s) », de l'extrait de *Ginkgo biloba* et/ou au moins de la cytidine-diphosphate-choline - et avantageusement les trois nutriments susvisés - représenterait plus de 1000 mg de nutriments, cette masse ne prenant pas en compte les additifs technologiques de dilution indispensables à une présentation finale du produit et qui représentent généralement une fraction comprise entre 30% et 80% de la composition finale. Si l'on intègre ces données, un complément alimentaire sous forme solide, comprenant l'association des trois nutriments mentionnés ci-dessus, aurait une masse de plus de 3000 mg et, par conséquent, un volume associé qui rendrait ledit complément alimentaire difficilement ingérable, affectant l'observance de la cure, tel que détaillé précédemment. Le fait de fractionner la dose à administrer en différentes sous-doses présente également des inconvénients, lesquels ont été également discutés supra.

Le recours à un complément alimentaire comprenant une source de magnésium à haute teneur en élément magnésium (de préférence sous forme stable), de l'extrait de *Ginkgo biloba* et/ou de la CDP-choline - avantageusement les deux - dans lequel ladite source de magnésium à haute teneur en élément magnésium (de préférence sous forme stable) comprend (et de préférence consiste en) au moins un mélange d'oxyde de magnésium et de carbonate de magnésium, autorise un apport important non seulement en extrait de *Ginkgo biloba* et/ou de CDP-choline (avantageusement en les deux) mais également en élément magnésium après ingestion dudit complément alimentaire, de préférence sous forme solide (avantageusement sous forme de comprimé), tout en utilisant des doses unitaires ayant une masse et un volume associé relativement faibles, parfaitement adaptées à une administration *per os* chez tout type de consommateur/sujet (même chez les consommateurs/sujets les plus âgés), et ce même pour des cures de plusieurs semaines voire plusieurs mois.

Par conséquent, l'invention concerne donc également un complément alimentaire (composition nutritionnelle) tel que défini précédemment comprenant en outre au moins un extrait de *Ginkgo biloba* et/ou au moins de la cytidine-diphosphate-choline (abrégée en CDP-choline ; également dénommée « citicoline »), avantageusement les deux.

Ledit au moins un extrait de *Ginkgo biloba* utilisé aux fins de la présente invention est préparé à partir des feuilles de cet arbre, lesquelles contiennent une variété de substances, à savoir notamment des flavonoïdes, des terpénoïdes, des lactones sesquiterpènes, des ginkgolides et des bilobalides. Cet extrait est de préférence sous forme solide, avantageusement sous forme pulvérulente. L'obtention d'un tel extrait de *Ginkgo biloba* est décrite dans la littérature scientifique. Des extraits de *Ginkgo biloba* convenant à la mise en oeuvre de la présente invention sont disponibles dans le commerce.

Selon un mode de réalisation préféré, le complément alimentaire (composition nutritionnelle) tel que défini précédemment, à savoir comprenant, outre le mélange d'oxyde de magnésium et de carbonate de magnésium, au moins un extrait de *Ginkgo biloba* et/ou au moins de la cytidine-diphosphate-choline, avantageusement les deux, comprend au moins 5 mg, de préférence au moins 30 mg, avantageusement au moins 40 mg, d'extrait de *Ginkgo Biloba* par unité de prise.

Concernant la cytidine-diphosphate-choline, ledit complément alimentaire comprend, selon un mode de réalisation préféré, au moins 50 mg, de préférence au moins 100 mg, avantageusement au moins 200 mg, de ce composé par unité de prise. Tout comme pour l'extrait de *Ginkgo biloba,* la cytidine-diphosphate-choline est disponible dans le commerce, par exemple sous forme de sodium de citicoline (« citicoline sodium » en langue anglaise), par exemple, sous forme de poudre/poudre cristalline de couleur blanche/blanc cassé.

De manière préférée, ledit complément alimentaire comprend au moins 250 mg, de préférence au moins 300 mg, avantageusement au moins 400 mg, de manière particulièrement préférée au moins 450 mg, du susdit mélange d'oxyde de magnésium et de carbonate de magnésium, par unité de prise.

De préférence, ledit complément alimentaire comprend en outre :
- au moins une vitamine, telle que la vitamine B5, de préférence dans une masse comprise entre environ 0,5 mg et environ 10 mg, préférablement comprise entre environ 0,7 mg et environ 5 mg, avantageusement comprise entre environ 0,8 mg et environ 1,2 mg, par unité de prise et/ou
- au moins un sel de zinc, tel que le glycinate de zinc, de préférence dans une masse comprise entre environ 0,5 mg et environ 5 mg, préférablement comprise entre environ 1 mg et environ 3 mg, avantageusement comprise entre environ 1,3 mg et environ 1,8 mg, par unité de prise ;
avantageusement ledit complément alimentaire comprenant ladite au moins une vitamine et ledit au moins un sel de zinc.

Selon un mode de réalisation particulièrement préféré, l'invention concerne un complément alimentaire administrable par voie orale à un individu humain ou animal, de préférence humain, ledit complément alimentaire comprenant, par unité de prise :
- au moins 250 mg, de préférence au moins 300 mg, avantageusement au moins 400 mg, de manière particulièrement préférée au moins 450 mg, d'un mélange d'oxyde de magnésium et de carbonate de magnésium, de préférence en tant que seule source de magnésium,
- au moins 5 mg, de préférence au moins 30 mg, avantageusement au moins 40 mg, d'extrait de *Ginkgo Biloba,* et
- au moins 50 mg, de préférence au moins 100 mg, avantageusement au moins 200 mg, de cytidine-diphosphate-choline.

Selon un mode de réalisation particulièrement préféré, le complément alimentaire selon la présente invention, comprenant au moins les deux ou trois nutriments susvisés (avantageusement au moins les trois), à savoir mélange d'oxyde de magnésium et de carbonate de magnésium, *Ginkgo biloba* et/ou CDP-choline, se présente sous forme solide, et de préférence sous forme de dose unitaire solide. Cette dose unitaire solide peut être sous forme de poudre, de granules, de gélule et, de manière particulièrement préférée, de comprimé. En effet, la présentation « comprimé » apparaît la plus appropriée, tel qu'expliqué précédemment. De préférence, ladite forme solide, préférablement sous forme de comprimé, a une masse inférieure à environ 1 g, de préférence inférieure à 1 g. Selon un mode de réalisation préféré, le complément alimentaire selon l'invention est obtenu par compression d'une composition comprenant au moins un mélange d'oxyde de magnésium et de carbonate de magnésium, de préférence en tant que seule source de magnésium, un extrait de *Ginkgo Biloba* et/ou de la CDP-choline (avantageusement les deux), ainsi qu'au moins un dérivé de cellulose comme additif de compression, ledit dérivé de cellulose étant avantageusement sélectionné dans le groupe constitué par les celluloses microcristallines séchées en jet d'air ou par atomisation tels que les produits EMCOCEL^{®} et VIVAPUR^{®} commercialisés par la société JRS ou encore les celluloses microcristallines de la gamme AVICEL^{®} séchées par atomisation ou par co-atomisation avec un additif de compression tel qu'un dérivé de phosphate de calcium, un polyol, ou une gomme végétale.

De manière optimale, le complément alimentaire selon la présente invention, comprenant les deux ou trois nutriments susvisés (avantageusement les trois), présente l'avantage d'être un complément alimentaire (composition nutritionnelle) hautement assimilable. Par « complément alimentaire (composition nutritionnelle) hautement assimilable » l'on entend, tel qu'indiqué supra, un complément alimentaire (composition nutritionnelle) dont les nutriments sont absorbés à plus de 80% au niveau du tube digestif.

Tel qu'indiqué précédemment, et sans être lié par la théorie, le caractère assimilable (hautement assimilable) du complément alimentaire selon l'invention comprenant les deux ou trois (avantageusement les trois) nutriments susvisés (mélange d'oxyde de magnésium et de carbonate de magnésium, *Ginkgo biloba* et/ou CDP-choline) semble dû à la cinétique de dissolution du complément alimentaire en milieu gastrique, garantissant aux consommateurs/sujets un apport suffisant en chacun des trois nutriments d'intérêt (et notamment en magnésium) tout en minimisant la masse/quantité du mélange oxyde de magnésium/carbonate de magnésium utilisé. En d'autres termes, le complément alimentaire selon la présente invention permet l'obtention d'un ratio teneur en nutriments (en particulier en magnésium)/masse du complément alimentaire particulièrement avantageux.

L'invention a également pour objet un procédé d'obtention de compléments alimentaires tels que définis précédemment sous forme de comprimé, ledit procédé comprenant les étapes suivantes :
a) introduction dans un mélangeur des constituants desdits compléments alimentaires à l'état pulvérulent ou préalablement granulé ;
b) mélange desdits constituants durant une durée adaptée à la taille du lot de fabrication envisagé ;
c) production des comprimés de masse souhaitée, de préférence de masse inférieure à environ 1 g, avantageusement de masse inférieure à 1 g, par compression du mélange obtenu à l'étape b) sur une presse de dimension adaptée ;
d) éventuellement contrôle et/ou conditionnement des comprimés obtenus à l'issue de l'étape c).

Selon un mode de réalisation particulier, ledit procédé comprend, entre les étapes b) et c), une étape de granulation humide et de séchage permettant la préparation de granulés, afin de faciliter l'étape de compression c).

Un autre objet de l'invention concerne l'utilisation (nutritionnelle) d'un complément alimentaire tel que défini précédemment ou d'un complément alimentaire susceptible d'être obtenu par le susdit procédé pour compléter le régime alimentaire normal d'un individu humain ou animal, de préférence d'un individu humain. En particulier, l'invention a pour objet l'utilisation (nutritionnelle) d'un complément alimentaire tel que défini précédemment ou d'un complément alimentaire susceptible d'être obtenu par le susdit procédé pour compléter le régime alimentaire normal d'un individu humain ou animal, de préférence humain, en magnésium. Cela permet en particulier :
- d'augmenter l'apport en magnésium ou combler un déficit en magnésium chez un individu humain ou animal, de préférence chez un individu humain, et/ou
- de prévenir, maintenir et/ou améliorer la vision et/ou tout ou partie des fonctions cognitives, en particulier la fonction de mémorisation, d'un individu humain ou animal, de préférence d'un individu humain.

En d'autres termes, l'invention concerne également l'utilisation (nutritionnelle) d'un complément alimentaire tel que défini précédemment ou d'un complément alimentaire susceptible d'être obtenu par le susdit procédé pour :
- augmenter l'apport en magnésium ou combler un déficit en magnésium chez un individu humain ou animal, de préférence chez un individu humain, et/ou
- prévenir, maintenir et/ou améliorer la vision et/ou tout ou partie des fonctions cognitives, en particulier la fonction de mémorisation, d'un individu humain ou animal, de préférence d'un individu humain.

Plus généralement, le complément alimentaire selon l'invention possède des effets neuroprotecteurs bénéfiques, en particulier chez les sujets âgés, et peut donc être utilisé dans la prévention des effets neurologiques associés au vieillissement.

Les exemples ci-après permettront de mieux appréhender la présente invention. Toutefois, ces exemples ne sont donnés qu'à titre illustratif et ne doivent en aucun cas être regardés comme limitant la portée de ladite invention d'une quelconque manière.

L'extrait de *Ginkgo biloba* utilisé aux fins des exemples présentés infra est le produit GINKGO PE (commercialisé par la société Naturex sous la référence 331744). Il s'agit d'un extrait sous forme pulvérulente obtenu à partir des feuilles de *Ginkgo biloba.*

Concernant la CDP-choline (citicoline) mentionnée dans les exemples qui suivent, il s'agit de sodium de citicoline (« citicoline sodium » en langue anglaise) obtenu auprès de la société britannique Cambridge Commodities Ltd (dont l'acronyme est « CCL »). Ce produit, commercialisé par la société CCL sous la référence P03294, se présente sous forme de poudre/poudre cristalline de couleur blanche/blanc cassé.

### Exemple 1

Un comprimé blanc d'une masse de 975 mg est obtenu par un procédé de compression directe à partir d'une composition comprenant les composés mentionnés ci-après, dans leurs quantités pondérales respectives, à savoir :

| | |
|---|---|
| - Extrait de *Ginkgo biloba* | 40 mg |
| - CDP-choline (citicoline) | 200 mg |
| - Oxyde de magnésium | 150 mg* |
| - Carbonate de magnésium | 300 mg** |
| - Additifs technologiques | qsp un comprimé de 975 mg |

| | |
|---|---|
| **Soit l'équivalent d'environ 90 mg de magnésium* ***Soit l'équivalent d'environ 60 mg de magnésium* | |

### Exemple 2

L'exemple 2 présente l'obtention d'un complément alimentaire selon l'invention sous forme de comprimé dans lequel l'oxyde de magnésium et le carbonate de magnésium sont présents dans des quantités pondérales différentes de celles présentées dans l'exemple 1 supra. Ainsi, un comprimé blanc d'une masse de 925 mg est également obtenu par un procédé de compression directe à partir d'une composition comprenant les composés mentionnés ci-après, dans leurs quantités pondérales respectives, à savoir :

| | |
|---|---|
| - Extrait de *Ginkgo biloba* | 40 mg |
| - CDP-choline (citicoline) | 200 mg |
| - Oxyde de magnésium | 175 mg* |
| - Carbonate de magnésium | 225 mg** |
| - Additifs technologiques | qsp un comprimé de 925 mg |

| | |
|---|---|
| **Soit l'équivalent d'environ 105 mg de magnésium* ***Soit l'équivalent d'environ 45 mg de magnésium* | |

### Exemple 3

L'exemple 3 présente l'obtention d'un complément alimentaire selon l'invention sous forme de comprimé dans lequel l'oxyde de magnésium et le carbonate de magnésium sont présents dans des quantités pondérales différentes de celles présentées dans les exemples 1 et 2 supra. Ainsi, un comprimé blanc d'une masse de 875 mg est également obtenu par un procédé de compression directe à partir d'une composition comprenant les composés mentionnés ci-après, dans leurs quantités pondérales respectives, à savoir :

| | |
|---|---|
| - Extrait de *Ginkgo biloba* | 40 mg |
| - CDP-choline (citicoline) | 200 mg |
| - Oxyde de magnésium | 200 mg* |
| - Carbonate de magnésium | 150 mg** |
| - Additifs technologiques | qsp un comprimé de 875 mg |

| | |
|---|---|
| **Soit l'équivalent d'environ 120 mg de magnésium* ***Soit l'équivalent d'environ 30 mg de magnésium* | |

### Exemple 4 (exemple comparatifs)

L'exemple 4 a pour but de préparer un comprimé de masse inférieure à 1 g par un procédé de compression directe, comme cela est le cas dans les exemples 1-3, en utilisant les mêmes quantités pondérales d'extrait de *Ginkgo biloba* et de CDP-choline mais en utilisant, en lieu et place du mélange d'oxyde de magnésium et de carbonate de magnésium, uniquement de l'oxyde de magnésium. Un comprimé blanc d'une masse inférieure à 1 g est donc obtenu par un procédé de compression directe, à partir d'une composition comprenant les composés mentionnés ci-après, dans leurs quantités pondérales respectives, à savoir :

| | |
|---|---|
| - Extrait de *Ginkgo biloba* | 40 mg |
| - CDP-choline (citicoline) | 200 mg |
| - Oxyde de magnésium | 250 mg* |
| - Additifs technologiques | qsp un comprimé inférieur à 1 g |

| | |
|---|---|
| **Soit l'équivalent d'environ 150 mg de magnésium* | |

### Exemple 5 (exemple comparatif)

L'exemple 5 a pour but de préparer un comprimé de masse inférieure à 1 g par un procédé de compression directe, comme cela est le cas dans les exemples 1-3, en utilisant les mêmes quantités pondérales d'extrait de *Ginkgo biloba* et de CDP-choline mais en utilisant, en lieu et place du mélange d'oxyde de magnésium et de carbonate de magnésium, uniquement du carbonate de magnésium. Un comprimé blanc d'une masse inférieure à 1 g cherche donc à être obtenu par un procédé de compression directe, à partir d'une composition comprenant les composés mentionnés ci-après, dans leurs quantités pondérales respectives, à savoir :

| | |
|---|---|
| - Extrait de *Ginkgo biloba* | 40 mg |
| - CDP-choline (citicoline) | 200 mg |
| - Carbonate de magnésium | 750 mg* |

| | |
|---|---|
| **Soit l'équivalent d'environ 150 mg de magnésium* | |

Il n'a malheureusement pas été possible de préparer un comprimé de masse inférieure à 1 g par compression directe, vraisemblablement du fait de la quantité trop importante de carbonate de magnésium dans la composition susvisée.

### Exemple 6 - dosage du magnésium dissous à partir des comprimés des exemples 1-4

Bien évidemment, dans la mesure où, tel qu'indiqué précédemment, il n'a pas été possible d'obtenir un comprimé de masse inférieure à 1 g en suivant l'enseignement de l'exemple 5 supra, seuls les comprimés préparés selon les enseignements respectifs des exemples 1-4 ont été testés.

Les comprimés préparés selon les enseignements respectifs des exemples 1 à 4 ont chacun été immergés dans 900 ml d'une solution de HCL 0,1 N, pH 1,6, dans un récipient avec agitateur magnétique chauffant (température : 37°C ; 60 rpm), afin de recréer *in vitro* les conditions de pH, de température et d'agitation rencontrées dans le milieu gastrique (conditions décrites dans la pharmacopée européenne), et ce durant 90 minutes.

Au terme des 90 minutes, 10 ml de chacune des solutions comprenant le comprimé d'intérêt sont prélevés puis filtrés sur filtre 0,45 µm ; la concentration en magnésium dissous étant ensuite analysée par ICP-AES (« Inductively Coupled Plasma Atomic Emission Spectroscopy » en langue anglaise). Les résultats sont présentés dans le Tableau 2 infra :

**Tableau 2 : dissolution du magnésium en milieu gastrique artificiel pour les comprimés des exemples 1-4**

| | Comprimé selon l'exemple 1 | Comprimé selon l'exemple 2 | Comprimé selon l'exemple 3 | Comprimé selon l'exemple 4 |
|---|---|---|---|---|
| % de magnésium dissous | >90% | >90% | >90% | <30% |

D'après les résultats montrés au sein du Tableau 2 supra, il apparaît que les comprimés obtenus par compression à partir des compositions décrites dans les exemples 1, 2 et 3, à savoir comprenant un mélange d'oxyde de magnésium et de carbonate de magnésium (dans des quantités pondérales variant d'un exemple à l'autre) permettent d'obtenir des taux de magnésium dissous supérieurs à 90%, conférant ainsi aux comprimés des exemples 1, 2 et 3 un caractère hautement assimilable.

A contrario, un comprimé obtenu selon l'enseignement de l'exemple 4, à savoir ayant une composition voisine de celle objet des exemples 1 à 3 (et une masse inférieure à 1 g) mais contenant uniquement un sel d'oxyde de magnésium en lieu et place d'un mélange d'oxyde de magnésium et de carbonate de magnésium donne lieu à une dissolution très faible du magnésium dans le milieu gastrique artificiel au terme des 90 minutes, à savoir un pourcentage de magnésium dissous inférieur à 30%. Il est évident qu'une telle composition n'est pas suffisamment (biologiquement) assimilable pour être utilisée efficacement dans le cadre d'une cure.

### Références bibliographiques

[1] Zhou H, Ma Y, Zhou Y, Liu Z, Wang K, Chen G.: Effects of magnésium sulfate on neuron apoptosis and expression of caspase-3, bax and bcl-2 after cerebral ischemia-reperfusion injury. Chin Med J (Engl). 2003 Oct;116(10):1532-4.
[2] Aydin B, Onol M, Hondur A, Kaya MG, Ozdemir H, Cengel A, Hasanreisoglu B.:The effect of oral magnésium therapy on visual field and ocular blood flow in normotensive glaucoma. Eur J Ophthalmol. 2010 Jan-Feb;20(1):131-5).
[3] Gaspar AZ, Gasser P, Flammer J.: The influence of magnésium on visual field and peripheral vasospasm in glaucoma. Ophthalmologica. 1995;209(1):11-3.
[4] Dietary Référence Intakes for Calcium, Phosphorous, Magnesium, Vitamin D, and Fluoride, 1997, Ces données sont le résultat d'un consensus entre les autorités canadiennes et américaines qui ont entrepris une démarche d'uniformisation des apports nutritionnels recommandés (ANR).
[5] Galan P, Preziosi P, et al. Dietary magnésium intake in a French adult population.Magnes Res. 1997 Dec;10(4):3218.
[6] Ford ES and Mokdad AH. Dietary magnésium intake in a national sample of U.S. adults. J Nutr. 2003;133:287982. Texte intégral : http://www.nutrition.org
[7] Khaled K A : "Formulation and évaluation of hydrochlorothiazide liquisolid tablets", SAUDI PHARMACEUTICAL JOURNAL, SAUDI PHARMACEUTICAL SOCIETY, RIYAD, SA, vol. 6, no. 1, 1 janvier 1998, pages 39-46
[8] Reynolds J E F ET AL : "MARTINDALE, THE EXTRA PHARMACOPOEIA, 28th edition", LONDON, PHARMACEUTICAL PRESS, GB, pages 1068-1070, 1 janvier 1982

## Revendications

1. Complément alimentaire non-effervescent administrable par voie orale à un individu humain ou animal, de préférence humain, ledit complément alimentaire comprenant un mélange d'oxyde de magnésium et de carbonate de magnésium, de préférence en tant que seule source de magnésium.

2. Complément alimentaire selon la revendication 1 comprenant au moins 10% en poids, de préférence au moins 20% en poids, avantageusement au moins 30% en poids, et de manière préférée au moins 40% en poids, du mélange d'oxyde de magnésium et de carbonate de magnésium par rapport au poids total du complément alimentaire.

3. Complément alimentaire selon la revendication 1 ou 2, ledit complément alimentaire étant sous forme solide, de préférence sous forme de dose unitaire, avantageusement sous forme de comprimé.

4. Complément alimentaire selon la revendication 3, ladite forme solide ayant une masse inférieure à environ 1 g, de préférence inférieure à 1 g.

5. Complément alimentaire selon la revendication 3 ou 4, ledit complément alimentaire comprenant :
- un ou plusieurs additif(s) technologique(s) de compression tel(s) qu'au moins un diluant, au moins un lubrifiant, au moins un agent d'écoulement et/ou au moins un désintégrant,
- éventuellement au moins une substance naturelle ou synthétique telle qu'au moins un colorant ou au moins un arôme.

6. Complément alimentaire selon l'une des revendications 1 à 5, dans lequel ledit mélange d'oxyde de magnésium et de carbonate de magnésium comprend au moins 20% en poids, de préférence plus de 20% en poids, préférablement au moins 30% en poids, avantageusement entre 30% et 75% en poids, et de manière particulièrement préférée entre 40% et 75% en poids, de carbonate de magnésium.

7. Complément alimentaire selon l'une des revendications 1 à 6, ledit complément alimentaire comprenant en outre au moins un extrait de *Ginkgo biloba* et/ou au moins de la cytidine-diphosphate-choline, avantageusement les deux.

8. Complément alimentaire selon la revendication 7, ledit complément alimentaire comprenant au moins 5 mg, de préférence au moins 30 mg, avantageusement au moins 40 mg, d'extrait de *Ginkgo biloba* par unité de prise.

9. Complément alimentaire selon la revendication 7 ou 8, ledit complément alimentaire comprenant au moins 50 mg, de préférence au moins 100 mg, avantageusement au moins 200 mg, de cytidine-diphosphate-choline par unité de prise.

10. Complément alimentaire selon l'une de revendications 1 à 9, ledit complément alimentaire comprenant au moins 250 mg, de préférence au moins 300 mg, avantageusement au moins 400 mg, de manière particulièrement préférée au moins 450 mg, dudit mélange d'oxyde de magnésium et de carbonate de magnésium, par unité de prise.

11. Complément alimentaire selon l'une des revendications 1 à 10, ledit complément alimentaire comprenant :
- au moins une vitamine, telle que la vitamine B5, de préférence dans une masse comprise entre environ 0,5 mg et environ 10 mg, préférablement comprise entre environ 0,7 mg et environ 5 mg, avantageusement comprise entre environ 0,8 mg et environ 1,2 mg, par unité de prise et/ou
- au moins un sel de zinc, tel que le glycinate de zinc, de préférence dans une masse comprise entre environ 0,5 mg et environ 5 mg, préférablement comprise entre environ 1 mg et environ 3 mg, avantageusement comprise entre environ 1,3 mg et environ 1,8 mg, par unité de prise ;
avantageusement ledit complément alimentaire comprenant ladite au moins une vitamine et ledit au moins un sel de zinc.

12. Procédé d'obtention de compléments alimentaires selon l'une des revendications 1 à 11, sous forme de comprimé, ledit procédé comprenant les étapes suivantes :
a) introduction dans un mélangeur des constituants desdits compléments alimentaires à l'état pulvérulent ou préalablement granulé ;
b) mélange desdits constituants durant une durée adaptée à la taille du lot de fabrication envisagé ;
c) production des comprimés de masse souhaitée, de préférence de masse inférieure à environ 1 g, avantageusement de masse inférieure à 1 g, par compression du mélange obtenu à l'étape b) sur une presse de dimension adaptée ;
d) éventuellement contrôle et/ou conditionnement des comprimés obtenus à l'issue de l'étape c).

13. Utilisation nutritionnelle d'un complément alimentaire selon l'une des revendications 1 à 11 ou d'un complément alimentaire susceptible d'être obtenu par le procédé selon la revendication 12 pour compléter le régime alimentaire normal d'un individu humain ou animal, de préférence d'un individu humain.

14. Utilisation nutritionnelle d'un complément alimentaire selon l'une des revendications 1 à 11 ou d'un complément alimentaire susceptible d'être obtenu par le procédé selon la revendication 12 pour :
- augmenter l'apport en magnésium ou combler un déficit en magnésium chez un individu humain ou animal, de préférence chez un individu humain, et/ou
- prévenir, maintenir et/ou améliorer la vision et/ou tout ou partie des fonctions cognitives, en particulier la fonction de mémorisation, d'un individu humain ou animal, de préférence d'un individu humain.
